# EUROPEAN PATENT APPLICATION

(11) **EP 1 477 111 A1**
(43) Date of publication of application: **17.11.2004**
(21) Application number: 04252822.4
(22) Date of filing: 14.05.2004
(51) Int. Cl.: A61B 5/00, A61B 5/0205

(54) **System for monitoring the condition of a person**

(30) Priority: 15.05.2003 JP 2003136902; 16.04.2004 JP 2004121688
(71) Applicant: Seiko Instruments Inc., Chiba-shi, Chiba (JP)
(72) Inventor: Meagawa, Kazuya, Mihama-Ku, Chiba-shi, Chiba (JP); Iijima, Ryuji, Mihama-Ku, Chiba-shi, Chiba (JP); Moriya, Koichi, Mihama-Ku, Chiba-shi, Chiba (JP)
(74) Representative: Sturt, Clifford Mark

(57) **Abstract**

A condition monitoring system that can change information to be provided in response to requests from a subject, is disclosed.

In a condition monitoring system including a living body information measuring terminal (11) that measures living body information (111), a living body information receiving terminal (12) having an analyzing means (122) for receiving a measurement result and analyzing a condition of a subject, and a monitoring terminal (13) that monitors the condition of the subject, a method of analysis processing performed by the analyzing means (122) is changed in response to requests from the subject.

## Description

The present invention relates to a condition monitoring system and a condition monitoring method for monitoring a condition of a subject.

Terminals and systems which detect an activity of a human and monitor a condition of the human have been conventionally known. For example, there are a technique for providing a pedometer that detects up-and-down motions of a human associated with the walking to count the number of steps, a technique for providing a calorie calculator that measures an amount of exercise and a heart rate and calculates calorie consumed from a result of the measurement and personal data such as height, weight, sex, and age inputted in advance, and a technique for measuring matters concerning amotion of a human and analyzing the motion of the human from a result of the measurement.

The problem with the prior art is that, in any of the above-mentioned techniques, a detected signal is subjected to processing specific to a terminal or system to be converted into a signal of different form from that of the detected signal, with the result that a subject can receive only those information which are restricted to the terminal or system used. While it is possible to provide multiple kinds of information to the subj ect by attaching multiple sensors to one terminal, this also causes such problems that the size and cost of the terminal increase accordingly or that even information unnecessary for a user is provided.

In order to solve the above problems, according to the present invention, there is provided a condition monitoring system, including: a living body information measuring terminal having living body information measuring means for measuring living body information of a subject, and measurement result transmitting means for transmitting a measurement result measured by the living body information measuring means; and a living body information receiving terminal having measurement result receiving means for receiving the measurement result transmitted from the living body information measuring terminal, analyzing means for analyzing a condition of the subject from the received measurement result, and storing means for storing the measurement result and an analysis result obtained by the analyzing means, the condition monitoring system being characterized in that a method of analysis processing for the measurement result to be performed by the analyzing means is changed in response to requests from the subject.

Here, in the condition monitoring system according to the present invention, one or more kinds of method of analysis processing are prepared for one kind of the measurement result, and one or more kinds of method of analysis processing are prepared for plural kinds of combination of the measurement result, and the method of analysis processing is changed in response to requests from the subject.

Further, the method of analysis processing is a method of performing analysis processing on a condition of the subject at a time when the subject performs measurement of living body information and/or a condition of the subject in a long term.

Further, kinds of the living body information measuring terminal and/or measurement conditions of the living body information measuring means can be changed together with the method of analysis processing.

The changing of the method of analysis processing is performed upon the request from the subject. The request includes changing of the method of analysis processing according to the measurement result, the analysis result, external information such as weather, a place, and a time at the time when the subject measures living body information, and a request from a specialist such as a doctor or a nurse.

Further, the condition monitoring system includes a control terminal, the control terminal setting an analysis program for realizing the method of analysis processing adapted to the request of the subject, which is performed by the analyzing means of the living body information receiving terminal, and transmitting the analysis program to the living body information receiving terminal to execute the analysis program.

Further, the living body information measuring terminal includes analysis method determining means for determining the methodof analysis processing in response to requests from the subject, and the living body information measuring terminal can transmit the method of analysis processing determined by the analysis method determining means to the control terminal to request a change of the method of analysis processing to be performed by the analyzing means of the living body information receiving terminal.

Further, the analysis result is notified to the subject by a display unit or the like provided in the living body information receiving terminal and/or by the control terminal sending the analysis result to a sending destination desired by the subject by a sending method desired by the subject.

Further, the control terminal includes charge information managing means for calculating a charge to be charged to the subject according to a method of analysis processing adapted to a request of the subject, kinds and the number of the living body information measuring terminals owned by the subject, and a method of notifying the subject of the analysis result, and for managing the charge, and the control terminal notifies the subject of information on the calculated charge that is to be charged to the subject.

Note that, as for the number of living body information measuring terminals described above, either single or plural living body information measuring terminals may be used. Further, living body information to be measured by the living body information measuring terminal is behavior and/or vital sign of the subject.

A living body information measuring terminal, a living body information receiving terminal, a condition monitoring method, and a recording medium recording the condition monitoring method according to the present invention are a living body information measuring terminal, a living body information receiving terminal, a condition monitoring method, and a recording medium recording the condition monitoring method that are used for realizing the condition monitoring system of the present invention.

Specifically, a living body information measuring terminal of the present invention includes : living body information measuring means for measuring living body information of a subj ect; measurement result transmitting means for transmitting a measurement result measuredby the living body informationmeasuringmeans; and analysis method determining means for determining a method of analysis processing in response to requests from the subject, the living body information measuring terminal being capable of transmitting the method of analysis processing determined by the analysis method determining means to an outside to request a change of a method of analysis processing to be performed by the analyzing means of the living body information receiving terminal.

Further, a living body information receiving terminal of the present invention includes: analyzing means for analyzing a condition of the subject from a measurement result measured by the living body information measuring means; and storing means for storing the measurement result and an analysis result obtained by the analyzing means, in which a method of analysis processing for the measurement result to be performed by the analyzing means is changed in response to requests from the subject. Further, the analysis result is notified to the subject.

Further, the living body information receiving terminal receives from the control terminal an analysis program for realizing a method of analysis processing adapted to a request of the subject and executes the analysis program to realize analysis processing adapted to the request of the subject.

Further, the living body information receiving terminal further receives from the control terminal a charge to be charged to the subject, the charge being determined according to a method of analysis processing adapted to a request of the subject, kinds and the number of the living body information measuring terminals owned by the subject, and a method of notifying the subject of the analysis result, and notifies the subject of the charge to be charged to the subject.

A condition monitoring method of the present invention includes: a step of measuring living body information of the subj ect by the living body information measuring means of the living body information measuring terminal; a step of receiving a measurement result, which is obtained in the step of measuring the living body information, by the measurement result receiving means of the living body information receiving terminal; a step of changing a method of analysis processing for analyzing the measurement result by the analyzing means of the living body information receiving terminal, in response to requests from the subject; and a step of analyzing the measurement result according to a method of analysis processing adapted to a request of the subject by the analyzing means of the living body information receiving terminal.

Here, the request from the subject for changing a method of analysis processing includes changing of the method of analysis processing according to the measurement result, the analysis result, external information such as weather, a place, and a time at the time when the subj ect measures living body information, and a request from a specialist such as a doctor or a nurse.

Further, the condition monitoring method includes a step of notifying the subject of the analysis result.

Further, the condition monitoring method includes: a step of calculating a charge to be charged to the subject according to a method of analysis processing adapted to a request of the subject, kinds and the number of the living body information measuring terminals owned by the subj ect, and a method of notifying the analysis result to the subject; and a step of notifying the subject of information on the charge to be charged to the subject.

Further, a computer readable recording medium of the present invention has recorded therein a program for causing a computer to execute a condition monitoring method, the condition monitoring method including: a step of receiving the measurement result from the living body information measuring terminal by the measurement result receiving means of the living body information receiving terminal; a step of changing a method of analysis processing for analyzing the measurement result, in response to requests from the subject; and a step of analyzing the measurement result according to a method of analysis processing adapted to a request of the subj ect by the analysis means of the living body information receiving terminal.

According to the present invention, the method of analysis processing to be performed by the analyzing means is changed in response to requests from a subject, whereby it is possible for the subject to select data to be obtained to thereby obtain only information necessary for the subject.

In addition, the analyzing means is provided in the living body information receiving terminal, making it possible to manufacture the living body information measurement terminal in a small size and inexpensively.

In addition, in particular, in the case in which the living body information measurement terminal has the analysis method determining means and the analysis method transmitting means, even if a subject temporarily has analysis contents that the subject wishes to confirm, a transmission request for an analysis program concerning an analysis method for the analysis contents can be made to the monitoring terminal immediately. Thus, the subj ect can obtain a desired analysis result without waiting.

In addition, the request from the subj ect for changing a method of analysis processing includes changing of the method of analysis processing according to a measurement result, an analysis result, external information such as weather, a place, and a time at the time when the subject measures living body information, or a request from a specialist such as a doctor or a nurse. Thus, even if the subject has fallen into an emergency situation, it is possible to deal with the emergency situation promptly. For example, "in the case in which an abnormality has been found in living body information, the method of analysis processingmaybe changed according to judgment by an observer (a specialist)'' is included in methods of analysis processing selectable by the subject, whereby, even if the subject has fallen into an emergency situation, it is made possible to deal with the emergency situation promptly without overlooking the emergency situation.

Further, in the case in which the monitoring terminal has charge information managing means, and the living body information receiving terminal has the charge information display means, information on a usage charge for the condition monitoring system changed to a subject, which is managed by the charge information managingmeans, can be displayed on the charge information displaying means. Thus, the subject can always confirm the usage charge for the conditionmonitoring system, a payment status of the usage charge, status of use of the conditionmonitoring system, and the like whenever desired.

Moreover, when an administrator of the condition monitoring system feeds back an analysis result to a subject or transmits a usage charge for the condition monitoring system and a payment status of the usage charge, status of use of the condition monitoring system, and the like to the living body information receiving terminal, it is possible to attach additional information such as information on the condition monitoring system and an advertisement thereto. Consequently, the subject can obtain useful information other than an analysis result of living body information of the subject.

Embodiments of the present invention will now be described by way of further example only and with reference to the accompanying drawings, in which:-
Fig. 1 is ablockdiagramshowingabasicstructureofacondition monitoring system;
Fig. 2 is a diagram showing storage contents of a monitoring terminal database;
Fig. 3 is a block diagram of a condition monitoring system in accordance with a second embodiment;
Fig. 4 is a flowchart showing an example of a data analysis method;
Fig. 5 is a block diagram showing a conditionmonitoring system;
Fig. 6 is a flowchart showing an example of a data analysis method;
Fig. 7 is a block diagram of a condition monitoring system in accordance with a third embodiment;
Fig. 8 is a diagram showing an example of display of the number of steps, a walking distance, and calorie consumed;
Fig. 9 is Storage contents of a monitoring terminal database; and
Fig. 10 is a diagram showing an example of display of a charge information display unit.

Preferred embodiments of the present invention will be hereinafter described in detail with reference to the drawings.

### First Embodiment

A basic condition monitoring system in accordance with a first embodiment of the present invention will be described.

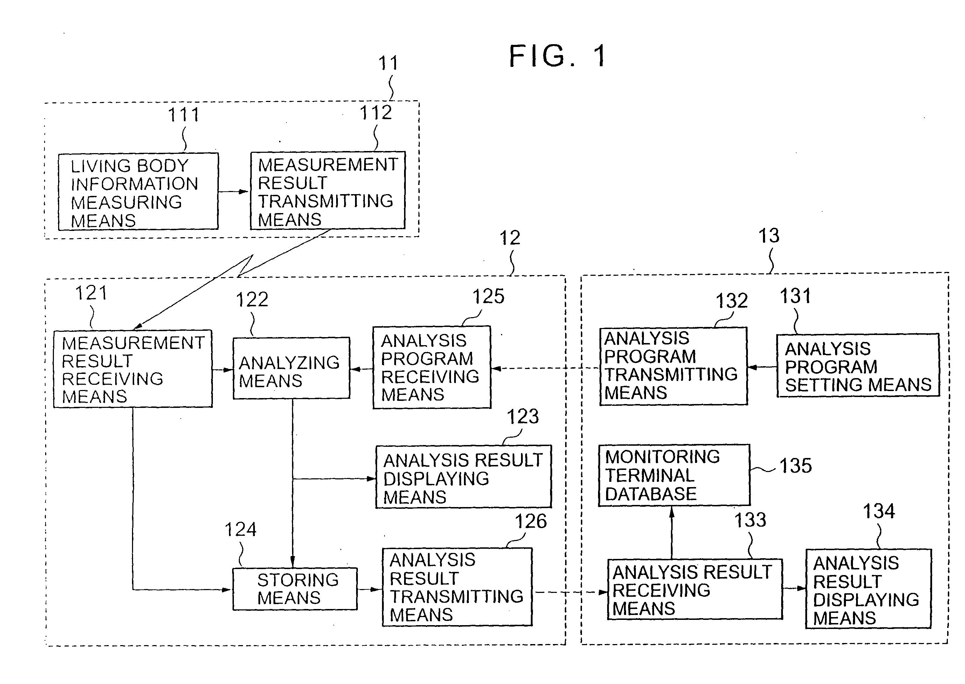

Fig. 1 is ablockdiagram showing abasic structure of a condition monitoring system in accordance with the present invention. The condition monitoring system is composed of a living body information measuring terminal 11, a living body information receiving terminal 12, and a monitoring terminal 13.

The living body information measuring terminal 11 includes: a living body information measuring means 111 for measuring living body information of a subject; and a measurement result transmitting means 112 for transmitting a measurement result.

The living body information receiving terminal 12 includes: a measurement result receiving means 121 for receiving the measurement result sent from the measurement result transmitting means 112; an analyzing means 122 for analyzing a condition of the subject using the measurement result received by the measurement result receiving means 121; an analysis result displaying means 123 for displaying an analysis result of the analyzing means 122; a storingmeans 124 for storing themeasurement result and the analysis result; an analysis program receiving means 125 for receiving an analysis program sent from the monitoring terminal 13; and an analysis result transmitting means 126 for transmitting the analysis result stored in the storing means to the monitoring terminal 13 using a public line.

The monitoring terminal 13 includes: an analysis program creating means 131 for creating an analysis program for attaining an analysis processing in analyzing a condition of the subject using the measurement result of the living body information measuring means 111 in the analyzingmeans 122; an analysis program transmitting means 132 for transmitting the analysis program to the living body information receiving terminal 12 using the public line; an analysis result receiving means 133 for receiving the analysis result sent from the analysis result transmitting means 126; an analysis result displaying means 134 for displaying the analysis result received by the analysis result receiving means 133; and a monitoring terminal database 135 in which individual information on subjects, analysis methods selected by the subj ects and analysis programs for attaining the analysis method, living body information and analysis results of the subjects, and the like are stored for each subject.

Here, the living body information to be measured by the living body information measuring means 111 includes vital sign such as a pulse, a heart rate, brain waves, and a body temperature and behavior such as a direction in which the subject has moved, an angle at which the subject has moved, a distance at which the subject has moved, a speed at which the subject has moved, and an acceleration at which the subject has moved. However, living body information is not limited to these pieces of information as long as the information is the vital sign or the behavior of the subject.

In addition, the analyzing means 122 performs analysis processing adapted to a request of the subject using the living body information measured by the living body information measuring means 111. For example, the analyzing means 122 not only performs calculation of a pulse rate and a heart rate from a pulse and a heart rate but also performs judgment on whether the subject is in a tension state while observing variation in the heart rate at an interval of R-R in response to requests from the subject. In addition, for example, the analyzing means 122 measures and displays a body temperature, and compares the body temperature with body temperatures of the subject in the past, which are saved in a database, and estimates a present physical condition of the subject in response to requests from the subject. Further, for example, the analyzing means 122 calculates the number of steps and calorie consumed from the behavior of the subject, and performs an analysis of a specific movement form focusing attention to a specific region in response to requests from the subject.

Moreover, the analyzing means 122 maybe automatically changed to various methods according to certain events if requested by the subject. The certain events include a measurement result of a specific living body information measuring terminal, a specific analysis result, external factors such as weather, a place, and a time at the time when measurement is performed, a changing instruction from the monitoring terminal and a changing instruction from the living body information measuring terminal, and whether or not a doctor acting as an observer makes a request.

In addition, communication methods between the measurement result transmitting means 112 and the measurement result receiving means 121 may be wired communication or wireless communication. However, the wireless communication is more preferable because measurement by the living body information measuring means is possible without preventing an activity of a subject. Any method may be adopted for the wireless communication, and for example, a short-range wireless data communication or Bluetooth can be used.

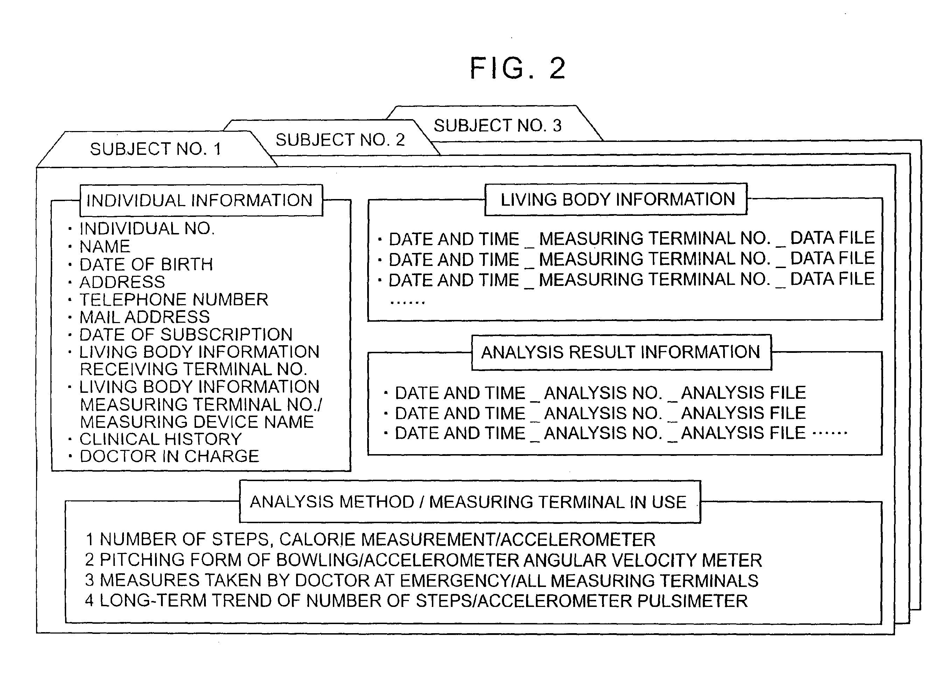

Fig. 2 shows specific storage contents of the monitoring terminal database 135. This condition monitoring system discloses these pieces of information when the subject makes a request to browse the information. In addition, in the case in which an analysis method selected by the subject is an analysis in a long-term span such as a statistical analysis of living body information, the monitoring terminal 13 performs an analysis based upon contents stored in a database by monitoring terminal analyzing means (not shown). This analysis result is transmitted to the living body information receiving means 12 by analysis result transmitting means (not shown) in the monitoring terminal and displayed on the analysis result displaying means 123.

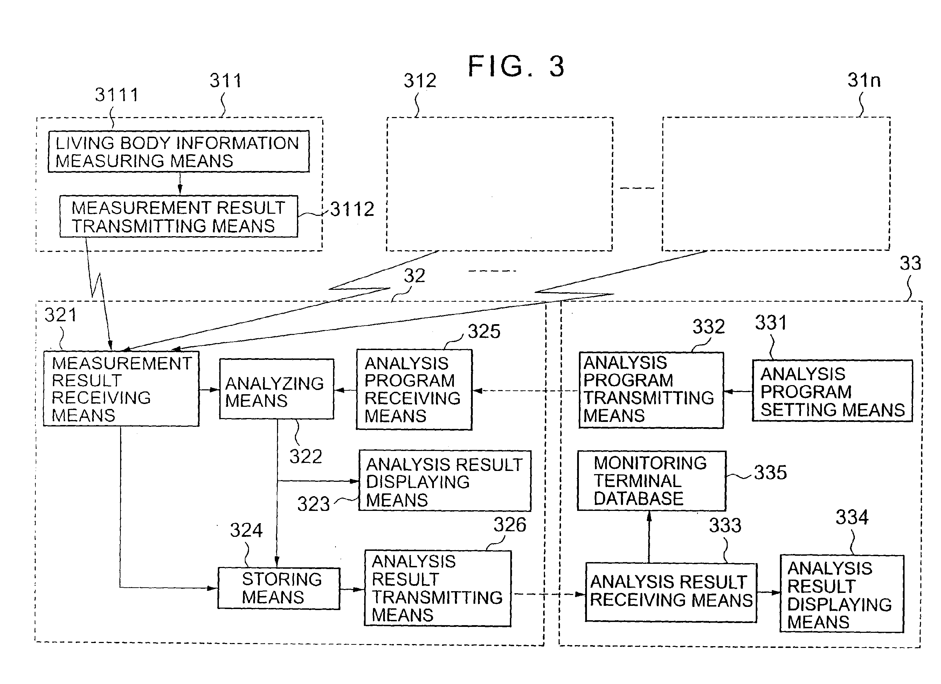

Referring next to Fig. 3, a second basic structure of the condition monitoring system in accordance with the present invention will be described.

The second basic structure includes: plural living body informationmeasuring terminals 311 to 31n; a livingbody information receiving terminal 32; and a control terminal 33. It is assumed there are n living body information measuring terminals.

The living body measuring terminal 311 includes: a living body information measuring means 3111 for measuring living body information of a subject; and a measurement result transmitting means 3112 for transmitting a measurement result to the living body information receiving terminal. The living body information measuring terminals 312 to 31n are terminals having the same structure as the living body information measuring terminal 311.

The living body information receiving terminal 32 includes: a measurement result receiving means 321 for receiving measurement results sent from the living body information measuring terminals 311 to 31n; an analyzing means 322 for analyzing a condition of the subject using the received plural measurement results; an analysis result displaying means 323 for displaying an analysis result of the analyzing means 322; a storing means 324 for storing the measurement result and the analysis result; an analysis program receiving means 325 for receiving an analysis program sent from the monitoring terminal 33; and an analysis result transmitting means 326 for transmitting the analysis result stpred in the storing means 324 to the monitoring terminal 33 using a public line.

The monitoring terminal 33 includes: an analysis program creating means 331 for creating an analysis program for attaining an analysis processing in analyzing a condition of the subject using a measurement result of the living body information measuring means in the analysis means; an analysis program transmitting means 332 for transmitting the analysis program to the living body information receiving terminal 32 using the public line; an analysis result receiving means 333 for receiving the analysis result sent from the analysis result transmitting means 326; an analysis result displaying means 334 for displaying the analysis result received in the analysis result receiving means 333; and a monitoring terminal database 335 in which individual information on subjects, analysis methods selected by the subjects, analysis programs for attaining the analysis methods, living body information and analysis results of the subjects, and the like are stored for each subject.

Here, the living body information measuring terminals 311 to 31n may be terminals, all of which measure living body information of the same type in such a manner that all the terminals measure behavior, or may be terminals, each of which measures different living body information in such a manner that a terminal measures behavior, another terminal measures pulse information, and another terminal measures a body temperature.

In addition, the analyzing means 322 performs analysis processing adapted to a request of the subject using the measurement results of the subject outputted from the living body information measuring terminals 311 to 31n. For example, the analyzing means 322 performs an analysis of a sleeping condition of the subject from information on a pulse, a heart rate, and brain waves. In addition, the analyzingmeans 322 calculates, for example, the number of steps and calorie consumed from the behavior of the subject, and in response to requests from the subject, performs an analysis of a specific movement form from plural pieces of behavior.

Further, the analyzing means 322 may be automatically changed to various methods according to certain events if requested by the subject. The certain events include a measurement result of a specific living body information measuring terminal, a specific analysis result, kinds and the number of pieces of living body information to be measured simultaneously, external factors such as weather, a place, and a time at the time when measurement is performed, a changing instruction from the monitoring terminal and a changing instruction from the living body information measuring terminal, and whether or not a doctor acting as an observer makes a request.

In addition, communication methods between the measurement result transmitting means 3112 and the measurement result receiving means 321 may be wired communication or wireless communication. However, the wireless communication is more preferable because measurement by the living body information measuring means is possible without preventing an activity of a subject. Any method may be adopted for the wireless communication, and for example, a short-range wireless data communication or Bluetooth can be used.

In addition, since contents of themonitoring terminal database 335 are identical with the contents of the monitoring terminal database 135 in Fig. 2, a description of the contents will be omitted.

The above-mentioned two types are conceivable as the basic structure of the condition monitoring system of the present invention. In the respective basic structures, it is possible to change a method of analysis processing, which is performed by the analyzing means provided in the living body information receiving terminal, in response to requests from the subject. For example, the condition monitoring system is designed such that, when the subject requests a method of analysis processing desired by the subject to the monitoring terminal in advance, the monitoring terminal transmits an analysis program adapted to the method of analysis processing to the living body information receiving terminal ownedby the subject. Since an analysis of living body information is performed in the living body information receiving terminal, functions of the living body information measuring terminal are only measurement of living body information and transmission of a measurement result, and it is possible to manufacture the living body information measuring terminal in a small size and inexpensively.

Next, specific operations of the condition monitoring system of the present invention will be described.

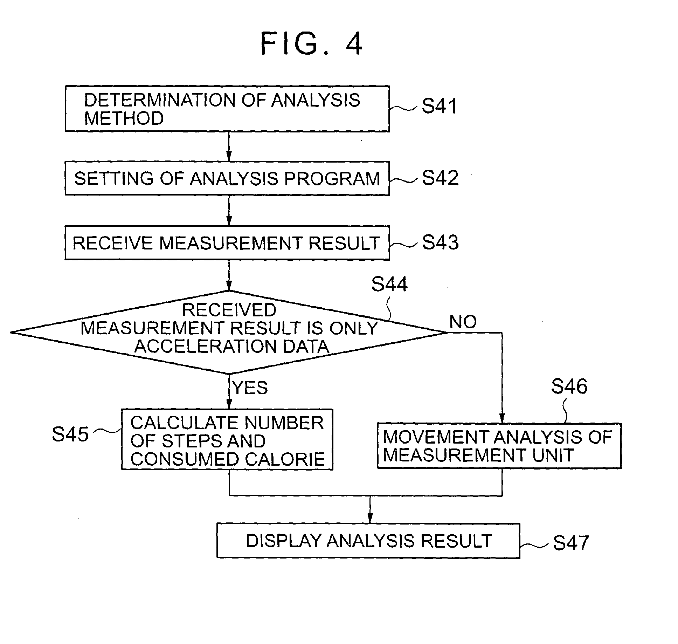

Fig. 4 is a flowchart showing an analysis method for living body information at the time when living body information of a subj ect is measured by plural living body information measuring terminals.

First, in installing this condition monitoring system, the subject selects a desired living body information measuring terminal out of various kinds of living body information measuring terminals. Here, it is assumed that the subj ect selects and owns an acceleration measuring terminal (denoted by 311) that is capable of measuring acceleration and an angular velocity measuring terminal (denoted by 312) that is capable of measuring angular velocity.

Next, subsequent to the selection of the living body information measuring terminal, the subject determines an analysis method that is performed using measuring results of the acceleration measuring terminal 311 and the angular velocity measuring terminal 312 (S41). The analysis method may be a fixed method such as one type of method for one living body information measuring terminal or may be automatically changed to various methods according to certain events. The certain events include a measurement result of a specific living body information measuring terminal, a specific analysis result, kinds and the number of pieces of living body information to be measured simultaneously, external factors such as weather, a place, and a time at the time when measurement is performed, a changing instruction from the monitoring terminal and a changing instruction from the living body information measuring terminal, and whether or not a doctor acting as an observer makes a request. Here, in the case in which the subject uses only the acceleration measuring terminal 311, the number of steps and calorie consumed are calculated. In the case in which the subject uses both the acceleration measuring terminal 311 and the angular velocity measuring terminal 312, movements of regions, where the respective terminals are attached, are analyzed. Here, if positions, where the acceleration measuring terminal 311 and the angular velocity measuring terminal 312 are attached, are set in advance, for example, an analysis with respect to specific movements such as a swing form of golf and a throwing form of bowling can be performed.

Next, an analysis program for executing the determined analysis method is set by the analysis program creating means 331 of the monitoring device 33 (S42). Then, the set analysis program is transmitted to the living body information receiving terminal 32 through a network. The living body information receiving terminal 32 receives the analysis program and stores the analysis program in a program storing unit (not shown), and then executes the program, making it possible to actually perform the analysis method determined by the subject.

Then, when the subject attaches the terminals to him/herself and starts the accelerationmeasuring terminal 311 and/or the angular velocity measuring terminal 312, the acceleration measuring terminal 311 and/or the angular velocity measuring terminal 312 starts measurement of living body information of the subject and transmits measurement results to the living body information receiving terminal 32 at a specific communication interval.

The living body information receiving terminal 32 receives a measurement result(s) from the acceleration measuring terminal 311 and/or the angular velocity measuring terminal 312 (S43) and performs an analysis of the received measurement result (S44). The analysis method follows contents of the analysis processing set in 542. In other words, in the case in which the received measurement result is a measurement result of the accelerationmeasuring terminal 311, the number of steps and calorie consumed of the subject are calculated (S45). In the case in which the received measurement results are measurement results of the acceleration measuring terminal 311 and the angular velocity measuring terminal 312, movements of regions, where the measurement terminal devices are attached, are analyzed from those measurement results (S46).

Results of the analysis in S45 and S46 are displayed on the analysis result displaying means 323 provided in the living body information receiving terminal 32 (S47). Consequently, it becomes possible for the subject to easily confirm the analysis result obtained by applying the analysis method desired by the subject to the living body information of the subject. For example, in the case in which only the accelerationmeasuring terminal 311 is attached, the subject can confirm the number of steps, a walking distance, and calorie consumed that are calculated from an amplitude and a period of measured acceleration.

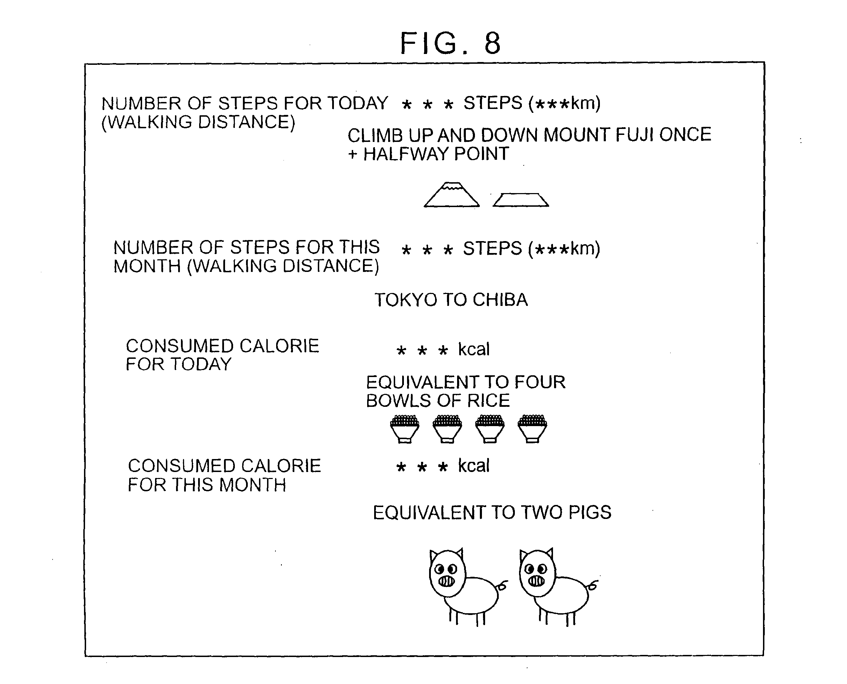

Fig. 8 shows an example of display of the number of steps, a walking distance, and calorie consumed. The number of steps (walking distance) and the calorie consumed on a measurement day as well as the number of steps (walking distance) and the calorie consumed in a measurement month are displayed on a display screen. The walking distances are converted into a distance of climbing the Mount Fuji and an arrival point from Tokyo to indicate which degrees of the distance and the arrival point the walking distances are equivalent to. In addition, the calorie consumed are converted into the number of rice bowls and the number of pigs to indicate which degrees of the numbers the calorie consumed are equivalent to.

Further, in the case in which the acceleration measuring terminal 311 and the angular velocity measuring terminal 312 are attached to the arm, the waist, or the like, the subject can confirm specific movements such as a swing form of golf or baseball and a pitching form of baseball or a throwing form of bowling that are analyzed on the basis of movements calculated from measured acceleration and angular velocity. Moreover, the monitoring terminal 33 stores reference information concerning movement forms of professional golfers and professional baseball players and movement forms of the subj ect in the past and transmits the reference information to the living body information receiving terminal 32 together with an analysis program, whereby the living body information receiving terminal 32 can compare the reference information and movement forms of the subject at present, analyze themovement forms, andprovide the subject of a result of the analysis. Consequently, the subject can compare the movement forms of the subject at present and the forms of the professional players and compare the forms of the subject at present and the forms of the subject in the past.

Note that the selection of the living body information measuring terminals 311 to 31n and the determination of an analysis method are performed when the subject installs this condition monitoring system. However, a change of a measuring terminal in use and a change of an analysis method in use may be performed while this condition monitoring system is operated.

In addition, it is possible to perform the determination and change of an analysis method using inputting means (not shown) provided in the living body information receiving terminal 32 other than the method of directly requesting the administrator of this condition monitoring system to perform the determination and change.

Further, concerning the analysis program used in the analyzing means 322 of the living body information receiving terminal 32, an example, in which the analysis program is transmitted from the monitoring terminal 33 to the living body information receiving terminal 32 through the network, is described. However, the present invention is not limited to this. For example, a form of directly downloading an analysis program stored in a storage medium to the living body information receiving terminal to change the analysis program may be adopted.

In this way, a method of analysis processing to be performed in analyzing means of the living body information receiving terminal is changed in response to requests from the subj ect, making it possible for the subject to obtain only information necessary for the subject from a result of living body information measurement of the subject.

### Second Embodiment

A condition monitoring system in accordance with a second embodiment of the present invention will be described.

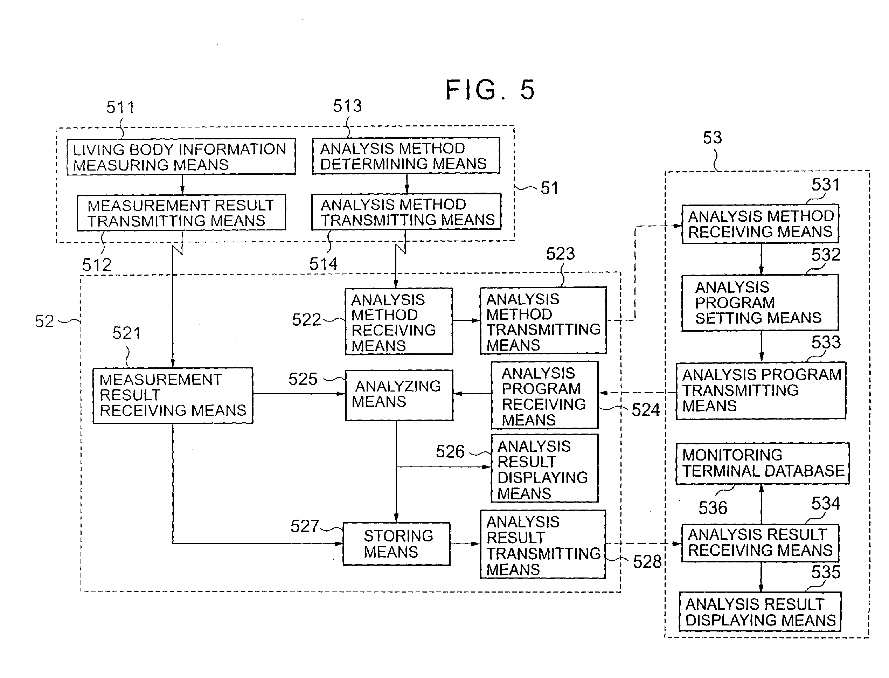

Fig. 5 is a block diagram of a condition monitoring system in accordance with the second embodiment of the present invention. The condition monitoring system is composed of a living body information measuring terminal 51, a living body information receiving terminal 52, and a monitoring terminal 53.

The living body information measuring terminal 51 includes: a living body information measuring means 511 for measuring living body information of a subject; a measurement result transmitting means 512 for transmitting a measurement result wirelessly; an analysis method determining means 513 for determining a method of analyzing the measured living body information; and an analysis method transmitting means 514 for transmitting the determined analysis method wirelessly.

Here, communication methods for the measurement result transmitting means 512 and the analysis method transmitting means 514 may be wired communication or wireless communication methods. However, the wireless communication is more preferable because measurement by living body information measuring means is possible without preventing an activity of a subject. Any method may be adopted for the wireless communication, and for example, a short-range wireless data communication or Bluetooth can be used.

In addition, it is unnecessary that the measurement result transmitting means 512 and the analysis method transmitting means 514 are separate transmitting means and the measurement result transmitting means 512 and the analysis method transmitting means 514 may be identical transmitting means.

The living body information receiving terminal 52 includes: a measurement result receiving means 521 for receiving the measurement result sent from the measurement result transmitting means 512; an analysis method receiving means 522 for receiving the analysis method sent from the analysis method transmitting means 514; an analysis method transmitting means 523 for transmitting the received analysis method to the monitoring terminal 53 using a public line; an analysis program receiving means 524 for receiving an analysis program to be transmitted from the monitoring terminal 53 using the public line; an analyzing means 525 for analyzing a condition of the subject from the measurement result, which is received by the measurement result receiving means 521, according to the received analysis program; an analysis result displaying means 526 for displaying an analysis result of the analyzing means 525; a storing means 527 for storing the measurement result and the analysis result; and an analysis result transmitting means 528 for transmitting the analysis result stored in the storing means 527 to the monitoring terminal 53 using the public line.

Here, it is unnecessary that the measurement result receiving means 521 and the analysis method receiving means 522 are separate receiving means and the measurement result receiving means 521 and the analysis method receiving means 522 may be identical receiving means.

In addition, it is unnecessary that the analysis method transmitting means 523 and the analysis result transmitting means 528 are separate transmitting means and the analysis method transmitting means 523 and the analysis result transmitting means 528 may be identical transmitting means.

The monitoring terminal 53 includes: an analysis method receiving means 531 for receiving the analysis method sent from the analysis method transmitting means; an analysis program setting means 532 for setting an analysis program for performing an analysis with the received analysis method; an analysis program transmitting means 533 for transmitting the set analysis program to the living body information receiving terminal 52 using the public line; an analysis result receiving means 534 for receiving the analysis result sent from the analysis result transmitting means 528; an analysis result displaying means 535 for displaying the analysis result received by the analysis result receiving means 534; and a monitoring terminal database 536 in which individual information on the subj ect, an analysis method selected by the subject and an analysis program for attaining the analysis method, living body information of the subject and an analysis result thereof, and the like are stored.

Here, it is unnecessary that the analysis method receiving means 531 and the analysis result receiving means 534 are separate receiving means and the analysis method receiving means 531 and the analysis result receiving means 534 may be identical receiving means.

In addition, since contents of themonitoring terminal database 536 are identical with the contents of the monitoring terminal database 135 in Fig. 2 of the first embodiment, a description of the contents will be omitted.

By adopting the structure of the system as described above, the subject can set a desired analysis method using the living body information measuring terminal 51 and can download a necessary analysis program to the living body information receiving terminal 52 from the monitoring device 53.

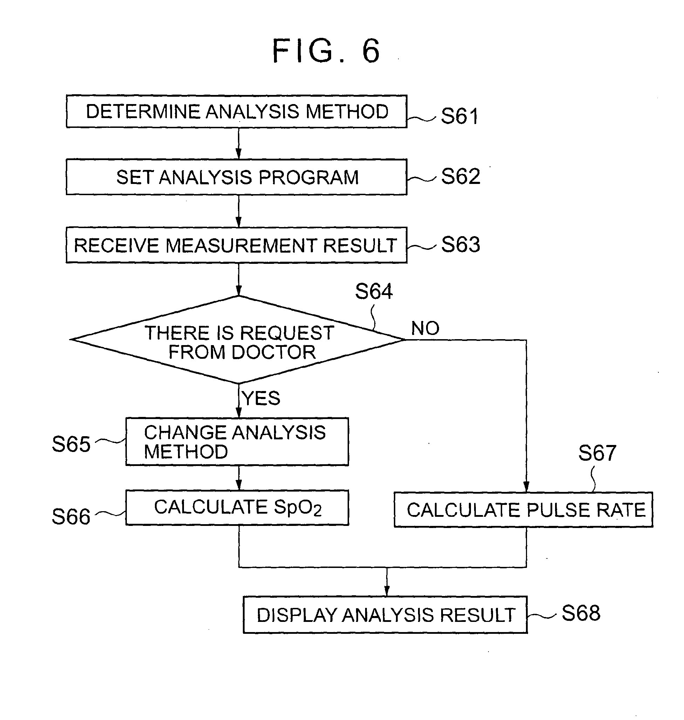

Fig. 6 is a flowchart showing an example of a data analysis method in measuring living body information using light.

First, a subject determines an analysis method for the living body information measuring terminal 51 that can measure living body information such as a pulse and SpO2 using light (S61). The analysis method may be a fixed method such as one type of method for one living body information measuring terminal or may be automatically changed to various methods according to certain events. The certain events include external factors such as a changing instruction from a monitoring terminal and a changing instruction from a living body information measuring terminal. Here, the analysis method is changed according to whether or not a doctor acting as an observer makes a request. If there is no request from the doctor, a pulse rate is calculated. If there is a request from the doctor, SpO2 is calculated. The determined analysis method is sent to the living body information receiving terminal 52 from the analysis method transmitting means 514 and further sent to the analysis method receiving means 531 of the monitoring terminal 53.

After the determined analysis method is received by the monitoring terminal 53, an analysis program for executing the determined analysis method is set by the analysis program setting means 532 (S62). The set analysis program is sent to the living body information receiving terminal 52 through a network. The living body information receiving terminal 52 receives the analysis program, stores the analysis program in a program storage unit (not shown) , and then executes the program, making it possible to actually execute the analysis method determined by the subject. The living body information measured by the living body information measuring terminal 51 is analyzed (S63). In that case, if there is a request from an observer such as a doctor monitoring the subject with the monitoring terminal 53 (S64), the analysis method is changed (S65) to perform an analysis. For example, if there is no request from the observer such as the doctor, the subject usually calculates only a pulse rate (S67) and displays a result of the calculation (S68). If there is a request from the doctor, oxygen saturation (SpO2) is calculated (S66) to display a result of the calculation (S68).

As a method of changing an analysis method in response to requests from an observer, for example, the observer directly requests a subject to change the analysis method by phone or the like, and the subject can change the analysis method for a living body information measuring terminal. Alternatively, the observer can access the living body information receiving terminal from a monitoring terminal through a public line to change the analysis method.

In addition, in this embodiment, the analysis method is changed in response to requests from the doctor. However, the subject can change the analysis method for himself/herself after taking a medicine and evaluate an effect of the medicine. In that case, the subject himself/herself sets the analysis method by the analysis method determining means 513 of the living body information measuring terminal 51.

In this way, a method of analysis processing to be performed in the analyzing means of the living body information receiving terminal is changed in response to requests from the subject, making it possible for the subject to obtain only information necessary for the subject from a result of living body information measurement of the subject.

In addition, since the living body information terminal includes the analysis method determining means and the analysis method transmitting means, even if there are analysis contents that the subject wishes to confirm temporarily, the subject can send a transmission request for an analysis program concerning an analysis method for the analysis contents to the monitoring terminal immediately. Then, the living body information receiving terminal receives the analysis program from the monitoring terminal and executes the analysis program. Thus, the subject canobtainadesired analysis result without waiting.

Further, "in the case in which an abnormality has been found in living body information, a processing method may be changed according to judgment of an observer" is included in methods for analysis processing to be selected by the subject, whereby, even if the subject has fallen into an emergency situation, it is possible to deal with the emergency situation with this condition monitoring system promptly without overlooking the emergency situation.

### Third Embodiment

A condition monitoring system in accordance with the present invention will be described as a third embodiment.

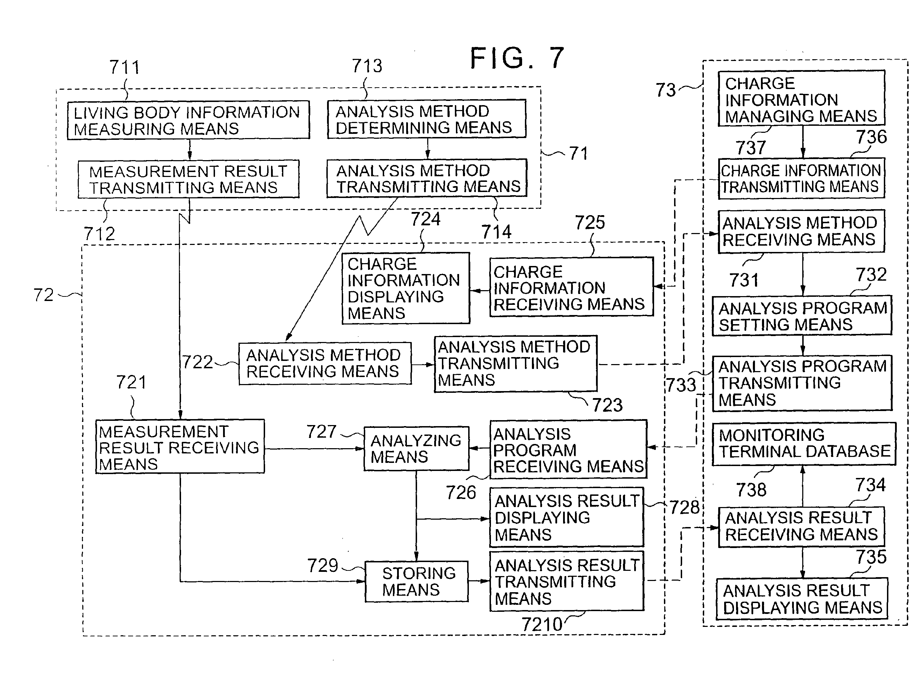

Fig. 7 is a block diagram of a condition monitoring system in accordance with the third embodiment of the present invention. The condition monitoring system is composed of a living body information measuring terminal 71, a living body information receiving terminal 72, and a monitoring terminal 73.

The living body information measuring terminal 71 includes: a living body information measuring means 711 for measuring living body information of a subject; a measurement result transmitting means 712 for transmitting a measurement result wirelessly; an analysis method determining means 713 for determining a method of analyzing the measured living body information; and an analysis method transmitting means 714 for transmitting the determined analysis method wirelessly.

Here, communication methods for the measurement result transmitting means 712 and the analysis method transmitting means 714 may be wired communication or wireless communication. However, the wireless communication is more preferable because measurement by living body information measuring means is possible without preventing an activity range of a subject. Any method may be adopted for the wireless communication, and for example, a short-range wireless data communication or Bluetooth can be used.

In addition, communication methods for the measurement result transmitting means 712 and the analysis method transmitting means 714 are not necessarily separate transmitting means but may be identical transmitting means.

The living body information receiving terminal 72 includes: a measurement result receiving means 721 for receiving the measurement result sent from the measurement result transmitting means 712; an analysis method receiving means 722 for receiving the analysis method sent from the analysis method transmitting means 714; an analysis method transmitting means 723 for transmitting the received analysis method to the monitoring terminal 73 using a public line; a charge information receiving means 725 for receiving information on a usage charge for the condition monitoring system of the subject to be transmitted from the monitoring terminal 73 using the public line; a charge information displaying means 724 for displaying the information received by the charge information receiving means 725 to the subject; an analysis program receiving means 726 for receiving an analysis program to be transmitted from the monitoring terminal 73 using the public line; an analyzing means 727 for analyzing a condition of the subject from the measurement result, which is received by the measurement result receiving means 721, according to the received analysis program; an analysis result displaying means 728 for displaying an analysis result of the analyzing means 727; a storing means 729 for storing the measurement result and the analysis result; and an analysis result transmitting means 7210 for transmitting the analysis result stored in the storing means 729 to the monitoring terminal 73 using the public line.

Here, the measurement result receiving means 721 and the analysis method receiving means 722 are not necessarily separate receiving means but may be identical receiving means.

In addition, the analysis method transmitting means 723 and the analysis result transmitting means 7210 are not necessarily separate transmitting means but may be identical transmitting means .

Further, the charge information receiving means 725 and the analysis program receiving means 726 are not necessarily separate receiving means but may be identical receiving means.

Moreover, the charge information displaying means 724 and the analysis result displaying means 728 are not necessarily separate displaying means but may be identical displaying means.

The monitoring terminal 73 includes: an analysis method receiving means 731 for receiving the analysis method sent from the analysis method transmitting means; an analysis program setting means 732 for setting an analysis program for performing an analysis with the received analysis method; an analysis program transmitting means 733 for transmitting the set analysis program to the living body information receiving terminal 72 using the public line; an analysis result receivingmeans 734 for receiving the analysis result sent from the analysis result transmitting means 721; an analysis result displaying means 735 for displaying the analysis result received by the analysis result receiving means 734; a charge information transmitting means 736 for transmitting information on a usage charge for the condition monitoring system of the subject to the living body information receiving means 72; a charge information managing means 737 for managing information on the usage charge based on status of use of the condition monitoring system by the subject; and a monitoring terminal database 738 in which individual information on the subject, an analysis method selected by the subject and an analysis program for attaining the analysis method, living body information of the subject and an analysis result thereof, a method of feeding back the analysis result to the subject and a feedback destination, information on payment of the usage charge for the condition monitoring system of the subject, and the like are stored.

Here, the charge information transmitting means 736 and the analysis program transmitting means 733 are not necessarily separate transmitting means but may be identical transmitting means.

In addition, the analysis method receiving means 731 and the analysis result receiving means 734 are not necessarily separate receiving means but may be identical receiving means.

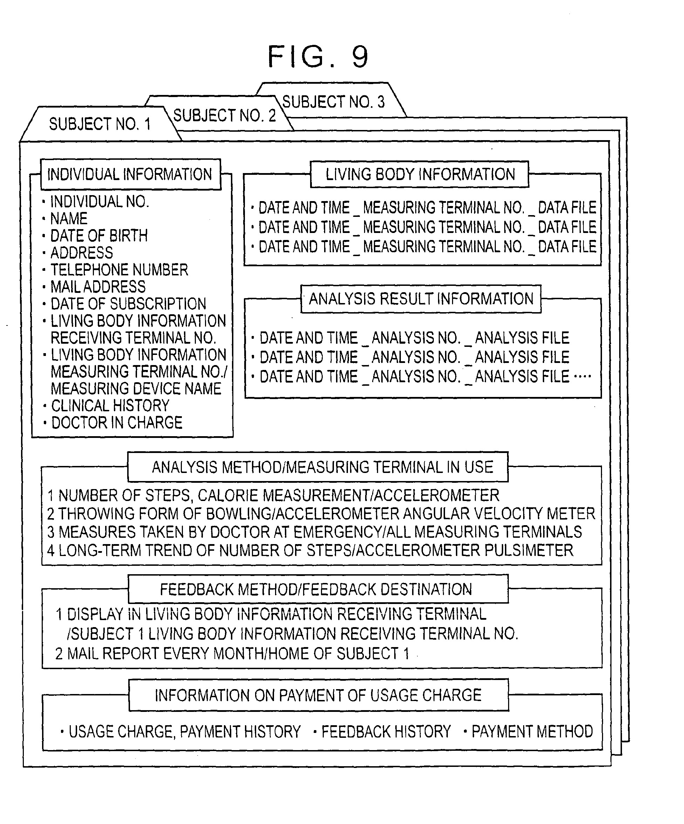

Fig. 9 shows specific storage contents of the monitoring terminal database 738. The contents include the monitoring terminal database 135 in Fig. 2 of the first embodiment added with a method of feeding back an analysis result to a subject and a feedback destination, and information on payment of a usage charge for the condition monitoring system of the subject.

As themethodof feedingbackthe analysis result to the subject, there are methods of displaying the analysis result on the analysis result displaying means 728 of the living body information receiving terminal 72 owned by the subject, transmitting analysis result information to a communication terminal owned by the subject, mailing the analysis result information to the subject, and the like. The method is selected by the subject in advance. In addition, the feedback destinations include the subject himself/herself, a relative of the subject, and a third party desired by the subject. The feedback destination is selected by the subject in advance.

The information on payment of a usage charge for the condition monitoring system of the subject includes a usage charge for the condition monitoring system of the subject, a method of payment of the usage charge for the condition monitoring systemof the subject, a history of payment of the usage charge for the condition monitoring system of the subject, and a history of feedback of an analysis result to the subject of the condition monitoring system. Here, the usage charge for the condition monitoring system is determined by the charge information managing means 737 taking into account kinds and the number of living body information measuring terminals owned by the subject, an analysis method selected by the subject, a feedback method selected by the subject, a feedback destination selected by the subject, and the like.

Note that the information included in the monitoring terminal database 738 such as information on payment of a usage charge for the condition monitoring system of the subject is designed so as to be transmitted to the living body information receiving terminal 72 by the charge information transmitting means 736 periodically or at a time desired by the subject.

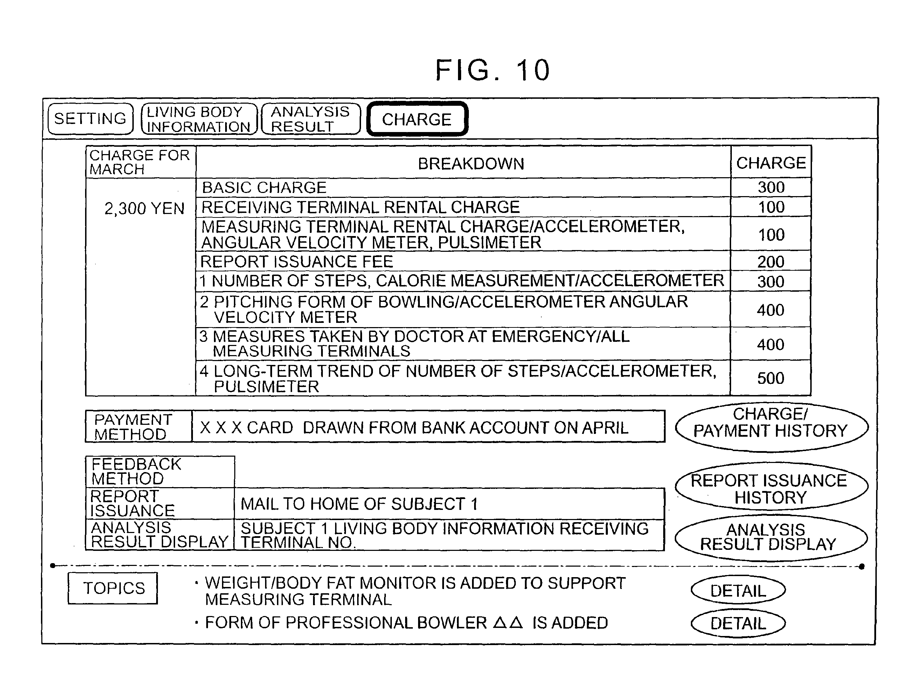

Fig. 10 shows an example of display of a charge information display unit that can be viewed on the charge displaying means 724. Fig. 10 shows a breakdown of a usage charge of the condition monitoring system of a subject for the month, a method of payment of the usage charge for the condition monitoring system of the subject, a method of feeding back an analysis result of the subject to the subject, and a feedback destination. In addition, a "charge payment history", a "report issuance history", an "analysis result display" shown in the right of the figure means that it is possible to perform display of a charge payment history, display of a report issuance history, and display of an analysis result according to an instruction by input from inputting means (not shown). Further, in the lower part of the figure, topics information in the condition monitoring system is displayed. In this way, it is also possible to attach additional information such as information on the condition monitoring system and an advertisement to the display and inform the subject of the additional information.

By adopting the structure of the system as described above, the subject can always confirm a usage charge for the condition monitoring system, a payment status of the usage charge, status of use of the condition monitoring system, and the like whenever desired.

In addition, an administrator of the condition monitoring system can obtain a profit through payment of the usage charge for the condition monitoring system by the subject.

Further, the administrator of the condition monitoring system is capable of attaching additional information such as information on the condition monitoring system and an advertisement when the administrator feeds back an analysis result to the subject and when the administrator transmits a usage charge for the condition monitoring system and a payment status of the usage charge, status of use of the condition monitoring system, and the like to the living body information receiving terminal 72. Consequently, the subject can obtain useful information other than an analysis result of living body information of the subject.

Note that it is also possible to record a program for realizing the functions of the living information receiving terminal in the embodiments of the present invention described above in a computer readable recording medium, cause an information device having a communication function to read the program recorded in this recording medium, and cause an information device to execute the program to thereby use the information device as a living body information receiving terminal.

## Claims

1. A condition monitoring system, comprising:
a living body information measuring terminal including:
living body information measuring means for measuring living body information of a subject; and
measurement result transmitting means for transmitting a measurement result measured by the living body information measuring means; and
a living body information receiving terminal comprising:
measurement result receiving means for receiving the measurement result transmitted from the living body information measuring terminal;
analyzing means for analyzing a condition of the subj ect from the received measurement result; and
storing means for storing the measurement result and an analysis result obtained by the analyzing means,
the condition monitoring system being **characterized in that** a method of analysis processing for the measurement result to be performed by the analyzing means is changed in response to requests from the subject.

2. A condition monitoring system according to claim 1,
wherein one or more kinds of method of analysis processing are prepared for one kind of the measurement result, and one or more kinds of method of analysis processing are prepared for plural kinds of combination of the measurement result, and wherein the method of analysis processing is changed in response to requests from the subject.

3. A condition monitoring system according to claim 1 or 2,
wherein the method of analysis processing comprises a method of performing analysis processing on a condition of the subject at a time when the subject performs measurement of living body information and/or a condition of the subject in a long term.

4. Aconditionmonitoringsystemaccordingto any one of claims 1 to 3, **characterized in that** kinds of the living body information measuring terminal and/or measurement conditions of the living body information measuring means are changed together with the method of analysis processing.

5. A condition monitoring system according to any one of claims 1 to 4,
wherein the request from the subj ect for changing of the method of analysis processing includes changing the method of analysis processing according to the measurement result, the analysis result, external information such as weather, a place, and a time at the time when the subject measures living body information, and a request from a specialist such as a doctor or a nurse.

6. Aconditionmonitoring systemaccording to anyone of claims 1 to 5, further comprising a control terminal, the condition monitoring system being **characterized in that** the control terminal comprises:
analysis program setting means for setting an analysis program for realizing the method of analysis processing adapted to the request of the subject which is performed by the analyzing means of the living body information receiving terminal; and
analysis program transmitting means for transmitting the analysis program to the living body information receiving terminal to cause the analyzing means to execute the analysis program.

7. A condition monitoring system according to claim 6, **characterized in that**:
the living body information measuring terminal comprises analysis method determining means for determining the method of analysis processing in response to requests from the subject; and
the living body information measuring terminal transmits the method of analysis processing determined by the analysis method determining means to the control terminal to request a change of the method of analysis processing to be performed by the analyzing means of the living body information receiving terminal.

8. A condition monitoring system according to claim 6,
wherein the analysis result is notified to the subject by a display unit or the like provided in the living body information receiving terminal and/or by the control terminal sending the analysis result to a sending destination desired by the subject by a sending method desired by the subject.

9. Aconditionmonitoringsystemaccordingtoanyoneof claims 6 to 8, **characterized in that**:
the control terminal comprises charge information managing means for calculating a charge to be charged to the subject according to a method of analysis processing adapted to a request of the subj ect, kinds and the number of the living body information measuring terminals owned by the subject, and a method of notifying the subject of the analysis result, and for managing the charge; and
the control terminal notifies the subject of information on the charge to be charged to the subject, which is calculated by the charge information managing means.

10. A condition monitoring system according to any one of claims 1 to 9, **characterized in that** there are a plurality of the living body information measuring terminals.

11. A condition monitoring system according to any one of claims 1 to 10, **characterized in that** living body information to be measured by the living body information measuring terminal is behavior and/or vital sign of the subject.

12. A living body information measuring terminal for implementing the condition monitoring system as claimed in claim 1, comprising analysis method determining means for determining a method of analysis processing in response to requests from the subject, the living body information measuring terminal being **characterized in that** the living body informationmeasuring terminal transmits the method of analysis processing determined by the analysis method determining means to an outside to request a change of a method of analysis processing to be performed by the analyzing means of the living body information receiving terminal.

13. A living body information receiving terminal for implementing the condition monitoring system as claimed in claim 1, **characterized in that** a method of analysis processing for the measurement result to be performed by the analyzing means is changed in response to requests from the subject.

14. A living body information receiving terminal according to claim 13, further comprising a function of notifying the subject of the analysis result.

15. The living body information receiving terminal according to claim 13 or 14, wherein:
the condition monitoring system further comprises a control terminal; and
the living body information receiving terminal receives from the control terminal an analysis program for realizing a method of analysis processing adapted to a request of the subject and executes the analysis program to realize analysis processing adapted to the request of the subject.

16. A living body information receiving terminal according to any one of claims 13 to 15,
wherein the livingbody information receiving terminal further receives from the control terminal a charge to be charged to the subject, the charge being determined according to a method of analysis processing adapted to a request of the subject, kinds and the number of the living body information measuring terminals owned by the subject, and a method of notifying the subject of the analysis result, and notifies the subject of the charge to be charged to the subject.

17. A condition monitoring method used in the condition monitoring system as claimed in claim 1, the condition monitoring method being **characterized by** comprising:
a step of measuring living body information of the subject by the living body information measuring means of the living body information measuring terminal;
a step of receiving a measurement result, which is obtained in the step of measuring the living body information, by the measurement result receiving means of the living body information receiving terminal;
a step of changing a method of analysis processing for analyzing the measurement result by the analyzing means of the living body information receiving terminal, in response to requests from the subject; and
a step of analyzing themeasurement result according to amethod of analysis processing adapted to a request of the subject by the analyzing means of the living body information receiving terminal.

18. A condition monitoring method according to claim 17,
wherein the request from the subject for changing a method of analysis processing, which is used in the step of changing a method of analysis processing for analyzing the measurement result by the analyzing means of the living body information receiving terminal, includes changing of the method of analysis processing according to the measurement result, the analysis result, external information such as weather, a place, and a time at the time when the subject measures living body information, and a request from a specialist such as a doctor or a nurse.

19. A condition monitoring method according to claim 17, further comprising a step of notifying the subject of the analysis result, which is obtained in the step of analyzing the measurement result according to a method of analysis processing adapted to a request of the subject by the analyzing means of the living body information receiving terminal.

20. A condition monitoring method according to any one of claims 17 to 19, further comprising:
a step of calculating a charge to be charged to the subject according to a method of analysis processing adapted to a request of the subject, kinds and the number of the living body information measuring terminals owned by the subject, and a method of notifying the analysis result to the subject; and
a step of notifying the subject of information on the charge to be charged to the subject.

21. A computer readable recording medium having recorded therein a program for causing a computer to execute a condition monitoring method used in the condition monitoring system as claimed in claim 1, the condition monitoring method being **characterized by** comprising:
a step of receiving the measurement result from the living body information measuring terminal by the measurement result receiving means of the living body information receiving terminal;
a step of changing a method of analysis processing for analyzing the measurement result, in response to requests from the subject; and
a step of analyzing the measurement result according to a method of analysis processing adapted to a request of the subject by the analysis means of the living body information receiving terminal.
